# EUROPEAN PATENT APPLICATION

(11) **EP 4 527 918 A1**
(43) Date of publication of application: **26.03.2025**
(21) Application number: 23807718.4
(22) Date of filing: 19.05.2023
(51) Int. Cl.: C12N 1/20, A23L 33/135, A61K 35/741, A61P 37/02, C12N 15/31

(54) **MICROORGANISM BELONGING TO GENUS AKKERMANSIA**

(30) Priority: 20.05.2022 JP 2022083151
(71) Applicant: Otsuka Pharmaceutical Co., Ltd., Tokyo 101-8535 (JP)
(72) Inventor: KOBAYASHI, Yodai, Osaka-shi, Osaka 540-0021 (JP); KOUDA, Noriyuki, Osaka-shi, Osaka 540-0021 (JP); INOUE, Shoichiro, Osaka-shi, Osaka 540-0021 (JP); KAWAHARA, Tomohiro, Osaka-shi, Osaka 540-0021 (JP); HAMURO, Koji, Osaka-shi, Osaka 540-0021 (JP); IBUKURO, Chika, Osaka-shi, Osaka 540-0021 (JP)
(74) Representative: Hoffmann Eitle
(86) International application number: PCT/JP2023/018734
(87) International publication number: WO 2023/224117

(57) **Abstract**

The present invention relates to a microorganism belonging to the genus *Akkermansia,* having a 16S rRNA gene comprising a nucleotide sequence etc. having homology of more than 98.0% to a nucleotide sequence set forth in SEQ ID NO: 3, having NOD2 agonistic activity, having assimilability to D-mannitol, being a Gram-negative, anaerobic bacterium with a spherical to short rod-shaped form, being negative in an oxidase test, and being positive in a catalase test, and a microorganism for activating NOD2, activating immunity, etc., comprising the microorganism as an active ingredient.

## Description

### [Technical Field]

The present invention relates to novel microorganisms belonging to the genus *Akkermansia,* and more specifically relates to such microorganism as well as compositions for activating NOD2, compositions for activating immunity, etc., which comprise the same as an active ingredient.

### [Background Art]

Various intestinal microorganisms gather in the human intestinal tract to form a complex ecosystem. This ecosystem is also called the intestinal microbiota, and it affects various physiological functions of humans, the host. Therefore, the species of microorganisms that inhabit the intestinal tract and their diversity are important for humans to live healthy and long lives.

In particular, regarding microorganisms belonging to the genus *Akkermansia,* which are a species of intestinal microorganism, it has been clarified that their abundance in the intestinal tract is effective for various conditions including metabolic disorders such as obesity and type 2 diabetes, and inflammatory diseases such as ulcerative colitis and appendicitis. In addition, in recent years, there has been a concept called the Brain-Gut-Microbiota Axis, and it has also been reported that the abundance of *Akkermansia* is related to the host's personality (level of sociability) (NPL 1).

Furthermore, an analysis of the intestinal microbiota of elderly people on Amami Oshima (Japan), where many individuals aged 100 or older reside (high centenarian rate), revealed that the proportion of *Akkermansia* and other bacteria was higher than that in those of the same age in other regions (NPL 2). In addition, it has been reported that the lifespan of progeroid model mice was extended when *Akkermansia* was administered to them (NPL 3), and these results suggest that the abundance of *Akkermansia* in the intestine has a positive effect on the lifespan of the host.

As described above, *Akkermansia* has recently attracted attention because it can affect the host's health, personality, etc., and ultimately extend the lifespan; however, only two species of microorganisms belonging to the genus *(Akkermansia muciniphila* and *Akkermansia glycaniphila)* have been identified.

### [Citation List]

### [Non Patent Literature]

[NPL 1] Katerina V-A Johnson, Hum Microb J.; "Gut microbiome composition and diversity are related to human personality traits", March 15, 2020, doi: 10.1016/j.humic.2019.100069.
[NPL 2] Hidetoshi Morita, Research report of Grant-in-Aid for Scientific Research, "Development of human longevity gut microbiology using the unique gut microbiota of centenarians in the Amami Islands as an indicator", June 23, 2021
[NPL 3] Clea Barcena et al., Nat Med.; "Healthspan and lifespan extension by fecal microbiota transplantation into progeroid mice", August 25, 2019, Volume 8, pp. 1234-1242, doi: 10.1038/s41591-019-0504-5.

### [Summary of Invention]

### [Technical Problem]

An object of the present invention is to isolate novel microorganisms belonging to the genus *Akkermansia* and to provide such microorganisms. A further object of the present invention is to clarify the effects of the microorganisms on the host and to provide a composition for improving the health condition of the host based on the effects.

### [Solution to Problem]

As a result of intensive studies to achieve the above objects, the present inventors have succeeded in detecting colony formation of microbial strains belonging to the genus *Akkermansia* (WON2089, WON2090, and WON2093) from the feces of 3 out of 5 subjects. Furthermore, as a result of determining and comparing the 16S rRNA sequences of these microbial strains, it was clarified that all of them carry the 16S rRNA gene composed of the same sequence (the sequence set forth in SEQ ID NO: 3).

As a result of BLAST+ analysis of the 16S rRNA sequences of the microorganisms against the rRNA/ITS reference strain database of the National Center for Biotechnology Information (hereinafter referred to as NCBI), the homology with known A. *muciniphila* (the sequence set forth in SEQ ID NO: 1; GenBank Accession No. AY271254) was 98.0%, and the homology with known A. *glycaniphila* was 94.0% (the sequence set forth in SEQ ID NO: 2; GenBank Accession No. KT254068). Furthermore, for WON2089, whole genome sequence analysis was determined, and based on the sequence, ANI analysis was performed by DFAST and compared with two bacterial strains of the genus *Akkermansia.* As a result, the ANI value was about 82.5% with known A. *muciniphila* and about 77.4% with known A. *glycaniphila.* Since the 95% ANI is the current species cutoff value, and the isolated microbial strains were below this, it was suggested that the isolated microbial strains could be a new species of *Akkermansia.*

Furthermore, in the results of the acid production test using API 20A, all of the microbial strains (WON2089, WON2090, and WON2093) showed growth on D-glucose, lactose, and D-mannose, as in the two known *Akkermansia* species, but unlike the two known species, growth on D-mannitol was also observed in all of the microbial strains.

Furthermore, when ligand activity was analyzed, NOD2 agonistic activity was not observed for the two known *Akkermansia* species, but the activity was observed in all of the microbial strains (WON2089, WON2090, and WON2093).

NOD2 is a type of pattern recognition receptor (PRR), and by activating such receptors, it becomes possible to detect invading pathogens and abnormal self-cells and eliminate them (so-called innate immunity and adaptive immunity).

Furthermore, regarding WON2089, the IgA secretion-promoting effect thereof was evaluated, and it was clarified that WON2089 has this effect; it was also found that the effect is at least twice as high as that of known *A. muciniphila* (type strain Muc, strain ATCC BAA-835), leading to the completion of the present invention.

That is, the present invention provides the following aspects.
[1] A microorganism belonging to the genus *Akkermansia,*
   having a 16S rRNA gene comprising a nucleotide sequence having homology of 98.0% or less to a nucleotide sequence set forth in SEQ ID NO: 1, homology of 94.0% or less to a nucleotide sequence set forth in SEQ ID NO: 2, or homology of more than 98.0% to a nucleotide sequence set forth in SEQ ID NO: 3, and
   having the following properties 1) to 4):
      1) having NOD2 agonistic activity,
      2) having assimilability to D-mannitol,
      3) being a Gram-negative, anaerobic bacterium with a spherical to short rod-shaped form, and
      4) being negative in an oxidase test and positive in a catalase test.
[2] A microorganism specified by accession number NITE BP-03622, NITE BP-03623 or NITE BP-03624.
[3] The microorganism according to [1], which further has an IgA secretion-promoting effect at least twice as high as that of type strain ATCC BAA-835 of *Akkermansia muciniphila.*
[4] A composition for activating immunity comprising the microorganism according to any one of [1] to [3] as an active ingredient.
[5] A composition for activating NOD2 comprising the microorganism according to any one of [1] to [3] as an active ingredient.
[6] A composition for promoting the secretion of IgA comprising the microorganism according to [2] or [3] as an active ingredient.

The present invention also relates to the use of the microorganism according to any one of [1] to [3] for producing a composition for activating immunity, a composition for activating NOD2, or a composition for promoting the secretion of IgA.

The present invention also relates to the use of the microorganism according to any one of [1] to [3] for activating immunity, for activating NOD2, or for promoting the secretion of IgA.

The present invention also relates to the microorganism according to any one of [1] to [3] for activating immunity, for activating NOD2, or for promoting the secretion of IgA.

The present invention also relates to a method for activating immunity, for activating NOD2, or for promoting the secretion of IgA, comprising administering an effective amount of the microorganism according to any one of [1] to [3] to a subject.

### [Advantageous Effects of Invention]

According to the present invention, it is possible to activate immunity via activation of NOD2 etc.

### [Brief Description of Drawings]

[Fig. 1] Fig. 1 is a phylogenetic tree created based on the 16S rRNA sequences of the microorganism of the present invention (WON2089), known *Akkermansia (A. muciniphila* and *A. glycaniphila),* and representative strains belonging to the phylum *Verrucomicrobia.*
[Fig. 2] Fig. 2 is a graph showing the results of analyzing the NOD2 agonistic activity of the microorganisms of the present invention (WON2089, WON2090, and WON2093) and known *Akkermansia (A. muciniphila* and A. *glycaniphila).*
[Fig. 3] Fig. 3 is a graph showing the results of analyzing the IgA secretion-promoting effect of the microorganism of the present invention (WON2089) and known *Akkermansia (A. muciniphila* (type strain Muc, strain ATCC BAA-835)).

### [Description of Embodiments]

### (Novel Microorganisms Belonging to the Genus Akkermansia)

As shown in Examples described later, the present inventors have succeeded in isolating microbial strains belonging to the genus *Akkermansia* (WON2089, WON2090, and WON2093) from human feces. It was clarified that these are microorganisms belonging to the same genus as, but different species from, the two known *Akkermansia* species *(A. muciniphila* and A. *glycaniphila),* and the possibility that they are new species was found. It has also been clarified that these novel microbial strains have different assimilability from known Akkermansia, and also have NOD2 agonistic activity, which is not detected in known *Akkermansia.*

Therefore, the novel microorganisms belonging to *Akkermansia* of the present invention (hereinafter also simply referred to as "the microorganisms of the present invention") relate to microorganisms
having a 16S rRNA gene comprising a nucleotide sequence having homology of 98.0% or less to a nucleotide sequence set forth in SEQ ID NO: 1, homology of 94.0% or less to a nucleotide sequence set forth in SEQ ID NO: 2, or homology of more than 98.0% to a nucleotide sequence set forth in SEQ ID NO: 3, and
having the following properties 1) to 4):
   1) having NOD2 agonistic activity,
   2) having assimilability to D-mannitol,
   3) being a Gram-negative, anaerobic bacterium with a spherical to short rod-shaped form, and
   4) being negative in an oxidase test and positive in a catalase test.

Generally, "microorganisms belonging to the genus *Akkermansia"* means anaerobic microorganisms (bacteria) belonging to the phylum *Verrucomicrobia,* class *Verrucomicrobiae,* order *Verrucomicrobiales,* family *Verrucomicrobiaceae,* genus *Akkermansia,* which are Gram-negative and have a spherical to short rod-shaped form. These microorganisms can also utilize mucin as a carbon and nitrogen source and an energy source. In addition, they have the properties of being negative in an oxidase test and positive in a catalase test. That is, microorganisms belonging to the genus *Akkermansia* are microorganisms that do not have cytochrome oxidase but do have catalase. Prior to the filing of the present application, two species of microorganisms belonging to the genus *Akkermansia* are known, namely A. *muciniphila (Akkermansia muciniphila)* and A. *glycaniphila (Akkermansia glycaniphila).*

The microorganisms of the present invention belong to the genus *Akkermansia,* but are at least different from the two known *Akkermansia* species. That is, the microorganisms are those whose 16S rRNA gene (16S rDNA) nucleotide sequence has homology of 98.0% or less (for example, 97.0% or less, 96.0% or less, 95.0% or less, 94.0% or less, 93.0% or less, 92.0% or less, 91.0% or less, 90.0% or less, 89.0% or less, 88.0% or less, or 87.0% or less) to that of A. *muciniphila* (the sequence set forth in SEQ ID NO: 1) or homology of 94.0% or less (for example, 93.0% or less, 92.0% or less, 91.0% or less, 90.0% or less, 89.0% or less, 88.0% or less, or 87.0% or less) to that of A. *glycaniphila* (the sequence set forth in SEQ ID NO: 2). The microorganisms of the present invention may also be microorganisms having a 16S rRNA gene comprising a nucleotide sequence having homology of more than 98.0% (for example, 98.5% or more, 98.7% or more, 99.0% or more, 99.5% or more, 99.7% or more, or 100.0%) to the nucleotide sequence set forth in SEQ ID NO: 3 (the nucleotide sequence of the 16S rRNA gene of WON2089, WON2090, and WON2093).

In the present invention, "homology" may mean similarity or identity. "Homology" may also mean identity in particular. Homology of nucleotide sequences can be determined using alignment programs such as BLAST (Basic Local Alignment Search Tool). For example, the homology of nucleotide sequences includes homology between sequences calculated using BLAST+ (National Center for Biotechnology Information) against the rRNA/ITS reference strain database of NCBI, and more specifically, the homology between sequences calculated using BLAST+ with default parameters (that is, using initial setting parameters). Further, in the present invention, homology is represented by a value obtained by rounding off the value calculated in this manner to the second decimal place.

The microorganisms of the present invention may be microorganisms that have an ANI (Average Nucleotide Identity) value of less than 95% (for example, 93% or less, 90% or less, 87% or less, 85% or less, 83% or less, or 80% or less) obtained in ANI analysis compared to the whole genome sequence of known *Akkermansia* (for example, A. *muciniphila).* Note that the ANI value can be obtained, for example, by using DFAST with default parameters (that is, initial setting parameters), as shown in Examples described later. The GC content in genomic DNA is preferably 56 to 57 mol % (particularly 56.8 mol %).

As shown in Examples described later, the microorganisms of the present invention also have properties that are partially different from those of the two known *Akkermansia* species in terms of assimilability. That is, the microorganisms of the present invention are characterized by having assimilability to at least D-mannitol (ability to assimilate D-mannitol), and preferably do not have assimilability to maltose (having the property of not being able to assimilate maltose). The microorganisms of the present invention may further have at least one of the following properties related to assimilation (for example, may further have 2 or more properties, 4 or more properties, 5 or more properties, 7 or more properties, 10 or more properties, 11 or more properties, 12 or more properties, 13 or more properties, or 14 properties).
Properties related to assimilation:
ability to assimilate D-glucose, ability to assimilate lactose, ability to assimilate D-mannose, inability to assimilate sucrose, inability to assimilate salicin, inability to assimilate D-xylose, inability to assimilate L-arabinose, inability to assimilate glycerin, inability to assimilate D-cellobiose, inability to assimilate D-melezitose, inability to assimilate D-raffinose, inability to assimilate D-sorbitol, inability to assimilate L-rhamnose, and inability to assimilate D-trehalose.

In the present invention, "assimilability" means sugar assimilability (ability to produce acid from sugar) in particular. Whether or not such assimilability is present can be determined by known acid production tests using, for example, an API system (API 20A), as shown in Examples described later.

As shown in Examples described later, the microorganisms of the present invention also have different properties from those of the two known *Akkermansia* species in terms of agonistic activity for pattern recognition receptors. That is, the microorganisms of the present invention are also characterized by having NOD2 agonistic activity, which these known ones do not have. Furthermore, the microorganisms of the present invention may have TLR2 agonistic activity at least 1.5 times (preferably 1.7 times or more, more preferably 2 times or more) higher and TLR4 agonistic activity at least 1.2 times (preferably 1.3 times or more, more preferably 1.5 times or more) higher than those of known *Akkermansia* (for example, A. *muciniphila* (strain ATCC BAA-835) and A. *glycaniphila* (strain DSM100705)).

In the present invention, "NOD2" is a protein also called nucleotide-binding oligomerization domain-containing protein 2, and if it is of human origin, it is a protein composed of the amino acid sequence set forth in, for example, NCBI Reference Sequence (RefSeq) ID: NP_001280486, NP_071445 or NP_001357395. "TLR2" is a protein also called Toll-like receptor 2, and if it is of human origin, it is a protein composed of the amino acid sequence set forth in, for example, RefSeq ID: NP_001305716, NP_001305718, NP_001305719, NP_001305720, NP_001305722, NP_001305724, NP_001305725 or NP_003255. "TLR4" is a protein also called Toll-like receptor 4, and if it is of human origin, it is a protein composed of the amino acid sequence set forth in, for example, RefSeq ID: NP_003257, NP_612564 or NP_612567.

"Agonistic activity" for such pattern recognition receptors means the property of microorganism-derived components to activate the receptors by being recognized (bound) by the receptors. Agonistic activity in the present invention includes not only activation of the receptors themselves (for example, multimerization), but also activation of downstream signal transduction caused by the activation (for example, binding of activated NOD2 to RICK, which is its downstream molecule, activation of the IKK complex by RICK, degradation of IκB, which is an inhibitory molecule of NF-κB, nuclear translocation of NF-κB released by the degradation, transcriptional activation by the translocation, and inflammatory response caused by the transcriptional activation). A person skilled in the art can quantitatively evaluate such agonistic activity by using, for example, cells into which pattern recognition receptor genes and reporter genes have been introduced (reporter assay system), as shown in Examples described later. In the present invention, a person skilled in the art can appropriately determine whether "having NOD2 agonistic activity" is present or absent depending on the evaluation system used; for example, for the reporter assay system shown in Examples described later, when the agonistic activity value obtained by culturing cells into which the reporter gene has been introduced in the presence of test microorganisms (10⁷ microorganisms/assay system) is significantly higher than the value obtained by culturing the cells in the absence of the microorganisms (for example, only medium), it can be determined that the test microorganisms have NOD2 agonistic activity. Note that a person skilled in the art can appropriately select and evaluate significance in such determination using a known statistical method depending on the number of target samples (N number), etc. For example, as shown in Examples described later, when the N number is 3 or more (for example, 4 or 5), it can be determined that there is a significant difference if the P value obtained by performing Student's t-test is less than 0.01. Furthermore, when the value obtained by culturing the cells in the presence of test microorganisms (10⁷ microorganisms/assay system) is at least one-fourth (preferably one-third or more) of the agonistic activity value obtained by culturing cells into which the reporter gene has been introduced in the presence of a positive control (for example, muramyl dipeptide (MDP) 10 µ g/assay system), it can also be determined that the test microorganisms have NOD2 agonistic activity.

As shown in Examples described later, the microorganisms of the present invention may also have a high IgA secretion-promoting effect. "IgA secretion" is the secretion of IgA (a substance also called IgA antibody or immunoglobulin A) from mucosa (especially intestinal mucosa in the present invention); "high" IgA secretion-promoting effect means that this effect is at least twice (preferably at least three times, more preferably at least four times) higher than that of known *Akkermansia* (for example, *A. muciniphila* (strain ATCC BAA-835)). A person skilled in the art can quantitatively evaluate such an IgA secretion-promoting effect by, for example, using a culture system containing Peyer's patches and immunologically detecting the amount of IgA secreted in the culture system (for example, by the ELISA method), as shown in Examples described later.

The microorganisms of the present invention may also have enzyme activities as shown in Examples described later. Specifically, they may have the activity of at least one enzyme selected from the group consisting of naphthol-AS-BI-phosphohydrolase, β-galactosidase, β-glucuronidase, and N-acetyl-β-glucosaminidase (for example, may have the activity of two, three, or four enzymes). They may not have the activity of at least one enzyme selected from the group consisting of alkaline phosphatase, esterase (C4), esterase lipase (C8), lipase (C14), leucine arylamidase, valine arylamidase, cystine arylamidase, trypsin, chymotrypsin, acid phosphatase, α-galactosidase, α-glucosidase, β-glucosidase, α-mannosidase, and α-fucosidase (for example, may not have the activity of at least five, seven, ten, twelve, or fifteen enzymes). Whether or not such enzyme activity is present can be determined by a known enzyme activity test using, for example, API ZYM, as shown in Examples described later.

The microorganisms of the present invention may also have a cellular fatty acid composition as shown in Examples described later. For example, they preferably contain 13:0 anteiso, which is not detected in the two known *Akkermansia* species. The proportion thereof is preferably 0.05% or more (for example, 0.06%, 0.07%, 0.08%, or 0.1%). On the other hand, they preferably do not contain Summed Feature 1, which is detected in the two known *Akkermansia* species (the proportion thereof is, for example, less than 0.1%, 0.05% or less, or 0.01% or less). The most major cellular fatty acid is 15:0 anteiso, and the proportion thereof is preferably 47% or more (for example, 50% or more, 52% or more, or 55% or more). Furthermore, it is preferable that they contain C15:0 at a proportion of 10% or less (for example, 7% or less or 5% or less), C15:0 iso 3OH at a proportion of 0.12% or more (for example, 0.15% or more or 0.2% or more), Sum In Feature 6 at a proportion of 0.25% or more (for example, 0.3% or more, 0.35% or more, or 0.4% or more), C17:0 iso at a proportion of 0.2% or more (for example, 0.25% or more or 0.3% or more), C17:0 anteiso at a proportion of 1% or more (for example, 1.2% or more, 1.5% or more, 1.7% or more, or 1.8% or more), C18:2 w6,9c at a proportion of 0.5% or more, C18:1 w9c at a proportion of 4% or more (for example, 5% or more or 5.5% or more), Sum In Feature 10 at a proportion of 0.4% or more, C18:0 at a proportion of 0.8% or more (for example, 0.9% or more), Sum In Feature 11 at a proportion of 0.1% or more (for example, 0.15% or more or 0.2% or more), or C17:0 anteiso 3OH at a proportion of 0.3% or more (for example, 0.4% or more, 0.5% or more, or 0.6% or more).

The cellular fatty acid composition can be analyzed, for example, according to the bacterial fatty acid composition analysis manual of the Sherlock Microbial Identification System, as shown in Examples described later. For the notation of fatty acids, refer to Table 8 below.

It is desirable that the microorganisms of the present invention exhibit growth conditions as shown in Examples described later. For example, regarding the growth temperature, in a 120-hour culture in modified GAM agar medium containing 0.1% mucin, they are preferably able to grow (proliferate) at 25 to 45°C, more preferably at 30 to 40°C, and still more preferably at 30 to 37°C. Furthermore, it is desirable that they are unable to grow at 20°C or less and/or 50°C or more under the same conditions. That is, the optimum growth temperature is desirably 30 to 40°C, and no growth is desirably confirmed at 20°C or less and/or 50°C or more. Regarding the growth pH, in a 120-hour culture at 37°C in modified GAM agar medium containing 0.1% mucin, they are preferably able to grow at pH 5.5 to 9.5, more preferably at pH 6.0 to 8.5, and still more preferably at pH 6.5 to 8.0. Furthermore, it is desirable that they are unable to grow at pH 5.0 or less and/or pH 10.0 or more under the same conditions. That is, the optimum pH is desirably 6.0 to 8.5, and no growth is desirably confirmed at pH 5.0 or less and/or pH 10.0 or more.

A person skilled in the art can appropriately prepare the microorganisms of the present invention based on known techniques. For example, by culturing gastrointestinal contents (feces, etc.) of humans, non-human mammals (for example, artiodactyls such as pigs and cattle, and rodents such as mice), poultry, reptiles, etc. in a known medium for *Akkermansia* culture (for example, Mucin Medium, mucin-containing modified GAM medium, or trypticase soy-containing medium) under anaerobic conditions as shown in Examples described later, the microorganisms of the present invention can be prepared.

Furthermore, as shown in Examples described later, WON2089, WON2090, and WON2093 are particularly preferred embodiments of the microorganisms of the present invention.

WON2089 was deposited with the NITE Patent Microorganisms Depositary of the National Institute of Technology and Evaluation (Room 122, 2-5-8 Kazusa-Kamatari, Kisarazu, Chiba 292-0818, Japan) on March 17, 2022 (identification label: WON2089, accession number: NITE BP-03622).

WON2090 was deposited with the NITE Patent Microorganisms Depositary of the National Institute of Technology and Evaluation on March 17, 2022 (identification label: WON2090, accession number: NITE BP-03623) .

WON2093 was deposited with the NITE Patent Microorganisms Depositary of the National Institute of Technology and Evaluation on March 17, 2022 (identification label: WON2093, accession number: NITE BP-03624).

Therefore, these bacterial strains can be obtained by applying to the depositary center.

The microorganisms of the present invention may also be microorganisms belonging to *Akkermansia* bred by mutation treatment, genetic recombination, selection of natural mutant strains, or the like, as long as they have at least NOD2 agonistic activity. Such a "mutant strain" means a strain that has been mutated to a specific microorganism (bacterial strain) by a method well known to those skilled in the art within a range that does not change its properties, a strain bred by selection of a natural mutant strain, or the like, or a strain that a person skilled in the art can confirm to be equivalent thereto. Further, the microorganisms of the present invention are not limited to the strains themselves that have been deposited or registered with a prescribed institution (hereinafter also referred to as "deposited strains"), and also include substantially equivalent strains (also referred to as "derived strains" or "induced strains"). Examples of the deposited strains of the present invention include WON2089, WON2090, and WON2093 described above. A "strain substantially equivalent to a deposited strain" means a strain belonging to *Akkermansia* and having at least NOD2 agonistic activity. A strain substantially equivalent to a deposited strain may be, for example, a derived strain whose parent strain is the deposited strain. Examples of derived strains include strains bred from a deposited strain and strains naturally occurring from a deposited strain.

The microorganisms of the present invention may be live bacteria or dead bacterial cells, or may be both live bacteria and dead bacterial cells. Examples of "dead bacterial cells" include heat-treated products, baked products, steamed products, radiation (γ-ray, etc.)-irradiated products, enzyme-treated products, drug (antibiotic, etc.)-treated products, chemical (formalin, etc.)-treated products, and ultrasonic wave-treated products. The form of the microorganisms of the present invention is not particularly limited and may be either liquid or solid, but the form is preferably a dry form (for example, bacterial powder) from the viewpoint of easy handling during production and storage. A dried product can be prepared by drying a suspension obtained by dispersing bacterial cells in a solvent (water or the like) . The drying method is not particularly limited, and examples thereof include spray drying and freeze drying. The sterilization method is not particularly limited, and examples thereof include retort sterilization, UHT sterilization, air sterilization, pressure sterilization, autoclaving, dry heat sterilization, flowing steam disinfection, electromagnetic wave sterilization, electron beam sterilization, high frequency sterilization, radiation sterilization, ultraviolet sterilization, ethylene oxide gas sterilization, hydrogen peroxide plasma sterilization, and chemical sterilization (alcohol sterilization, formalin fixation, and electrolytic water treatment). In some cases, before or after sterilization treatment by heating, etc., or before or after drying treatment, surfactant treatment, grinding/pulverization treatment, enzyme treatment, fractionation treatment, etc. may be performed, and products obtained by these treatments can also be included in the composition of the present invention.

Furthermore, the present invention may be provided not only in the form of the aforementioned bacterial cells but also in the form of constituents of the microorganisms, that is, components of the microorganisms themselves (so-called bacterial cell components, for example, amino acids, peptides, nucleic acids, sugars, lipids, vitamins, hormones, and complexes thereof, and further complexes thereof with phosphorus, sulfur, and metal elements, which constitute the microorganisms).

Furthermore, the present invention may also be in the form of a culture of the microorganisms of the present invention. The culture may be one containing the microorganisms (a culture solution, solid medium, etc., containing the proliferated microorganisms), and the form of the culture is not particularly limited and may be either liquid or solid, but the form is preferably a dry form (for example, a culture-dried product) from the viewpoint of easy handling during production and storage. Note that a person skilled in the art can appropriately prepare such culture-dried products using the above-described drying method.

### (Composition)

As shown in Examples described later, the microorganisms of the present invention described above have at least NOD2 agonistic activity. Therefore, the present invention provides a composition for activating NOD2 in a subject, the composition comprising the microorganisms of the present invention as an active ingredient.

Furthermore, the microorganisms of the present invention described above may also have a high IgA secretion-promoting effect. Therefore, the present invention provides a composition for promoting IgA secretion in a subject, the composition comprising the microorganisms of the present invention as an active ingredient.

NOD2 is a type of pattern recognition receptor, and by activating such receptors, it becomes possible to detect invading pathogens and abnormal self-cells and eliminate them (innate immunity and adaptive immunity). Therefore, the present invention provides a composition for activating innate immunity and/or adaptive immunity in a subject, the composition comprising the microorganisms of the present invention as an active ingredient.

Furthermore, IgA secreted in mucosal tissues prevents the invasion of pathogens, etc. on the surface of the tissues (so-called mucosal immunity). Therefore, the present invention provides a composition for activating mucosal immunity in a subject, the composition comprising the microorganisms of the present invention as an active ingredient.

Additionally, as described above, the present invention provides a composition for activating immunity in a subject, the composition comprising the microorganisms of the present invention as an active ingredient.

The microorganisms of the present invention contained as an active ingredient in such a composition may be not only the bacterial cells described above (live bacteria and dead bacterial cells) but also bacterial cell components and cultures thereof. For example, peptidoglycan derived from the microorganisms of the present invention (preferably peptidoglycan having an MDP-like structure) is suitable as an active ingredient of the composition for activating NOD2. In addition to the peptidoglycan, lipoteichoic acid, lipoprotein, lipoarabinomannan, zymosan, lipopolysaccharide (LPS), and lipid A are also suitable examples of active ingredients of the composition for activating innate immunity and/or adaptive immunity, from the viewpoint that they activate TLR2 or TLR4. Suitable examples of active ingredients of the composition for promoting IgA secretion include Pam₃CSK₄, lipoteichoic acid, and LPS.

The composition of the present invention may be in the form of a pharmaceutical composition (pharmaceutical product, quasi-drug, etc.), food composition (food and/or beverage, animal feed, etc.), or reagent used in cell experiments, model animal experiments, etc. The composition of the present invention can be formulated by a known pharmaceutical method depending on the form of the microorganisms of the present invention, which are the active ingredient. For example, capsules, tablets, pills, liquids, powders, granules, fine granules, film-coated preparations, pellets, troches, sublingual tablets, chewable tablets, buccal tablets, pastes, syrups, suspensions, elixirs, emulsions, liniments, ointments, plasters, cataplasms, transdermal preparations, lotions, inhalants, aerosols, injections, suppositories, etc. can be used orally or parenterally, but oral administration is desirable from the viewpoint of non-invasive and easy ingestion.

In these formulations, pharmacologically or food and/or beverage acceptable carriers can be appropriately combined; specifically, these include physiological saline, sterile water, media, vegetable oils, solvents, excipients, bases, emulsifiers, suspending agents, surfactants, stabilizers, flavoring agents, fragrances, vehicles, preservatives, binders, diluents, isotonic agents, soothing agents, bulking agents, disintegrating agents, buffering agents, coating agents, lubricants, coloring agents, sweeteners, viscous agents, flavor modifiers, solubilizers, or other additives and the like.

In view of the fact that the microorganisms of the present invention are intestinal microorganisms, particularly in preparations intended for oral administration, they may be combined with a composition that enables efficient delivery of the microorganisms of the present invention to the intestinal tract. The composition that enables such delivery to the intestinal tract is not particularly limited, and a known composition can be appropriately adopted; examples thereof include pH-sensitive compositions, compositions that suppress release until the intestinal tract (cellulose-based polymers, acrylic acid polymers and copolymers, vinyl acid polymers and copolymers, etc.), bioadhesive compositions that specifically adhere to the intestinal mucosa (for example, polymers described in US Patent No. 6368586), protease inhibitor-containing compositions, and compositions that are specifically decomposed by intestinal enzymes.

When the composition of the present invention is used as a pharmaceutical composition, it may be used in combination with a known substance used for activating immunity, etc. Examples of such known substances include probiotics such as lactic acid bacteria and bifidobacteria, and prebiotics.

When the composition of the present invention is used as a food composition, it can be, for example, a health food, functional food, food for specified health use, food with nutrient function claims, food with function claims, dietary supplement, food for patients, or animal feed. Functional foods are usually classified into four types: probiotics, biogenics, prebiotics, and synbiotics based on their mechanism of action, and the present invention can take the form of probiotics, biogenics, synbiotics, or prebiotics.

The food and/or beverage of the present invention can be ingested as a composition as described above, and can also be ingested as various foods and/or beverages. Specific examples of foods and/or beverages include liquid foods such as functional beverages, drink preparations, jelly-like beverages, soft drinks, milk beverages, tea beverages, alcoholic beverages, and soups; fermented foods and fermented beverages such as yogurt and drinking yogurt; products containing oils and fats such as edible oil, dressing, mayonnaise, and margarine; carbohydrate-containing foods such as cooked rice, noodles, and bread; livestock processed foods such as ham and sausage; marine processed foods such as kamaboko, dried fish, and salted fish guts; vegetable processed foods such as pickles; semi-solid foods such as jelly; fermented foods such as miso; and various confectioneries such as Western confectioneries, Japanese confectioneries, candies, gums, gummies, frozen desserts, and ice confections; retort products such as curry, thick starchy sauce, and Chinese soups; instant foods such as instant soups and instant miso soup, and microwave-ready foods. Furthermore, powdered, granulated, tableted, encapsulated, liquid, paste, or jelly-like health foods and/or beverages are also included. Note that the production of foods and/or beverages in the present invention can be carried out by production techniques known in the art. One or more ingredients effective for activating immunity etc. (for example, other intestinal microorganisms or ingredients effective for the proliferation of the microorganisms of the present invention in the intestinal tract etc.) may be added to the food and/or beverage. Furthermore, by combining it with other ingredients that exhibit functions other than such activation etc. or other functional foods, it may also be a multifunctional food and/or beverage.

The "other intestinal microorganisms" are not particularly limited, and examples thereof include probiotics such as lactic acid bacteria and bifidobacteria. Examples of the "ingredients effective for the proliferation of the microorganisms of the present invention in the intestinal tract etc." include N-acetyl-D-glucosamine, N-acetyl-D-galactosamine, and polyphenol.

Furthermore, the food and/or beverage composition proposed in the present invention can also be provided and sold as a food and/or beverage with health uses. The act of "indication" includes all acts that notify the consumer of the uses, and may be an expression that allows direct recognition of the uses, or an expression that evokes or analogizes the uses, attached to the composition itself, or an expression attached to a container, packaging material, or package insert containing the composition. Furthermore, the "indication" may also be a display or advertisement stating that the composition of the present invention is effective as information related to the composition of the present invention, in the forms of leaflets, pamphlets, pops, catalogs, posters, books, storage media such as DVDs, electronic bulletin boards, and advertisements such as those on the Internet.

### <Method for Activating Immunity etc.>

The present invention also provides a method for activating NOD2, a method for promoting IgA secretion, or a method for activating immunity in a subject, the methods comprising administering the microorganisms of the present invention or a composition containing the microorganisms as an active ingredient (the composition of the present invention described above) to the subject or allowing the subject to ingest the same. Examples of "immunity" to be activated in the present invention include, as described above, innate immunity, adaptive immunity, and mucosal immunity, and more specifically, elimination of pathogenic bacteria etc., induction of production of antimicrobial peptides (α-defensin etc.), promotion of T helper cell differentiation, and control of intestinal bacteria.

The "subject" for activating immunity etc. in the present invention is an animal including a human. Animals other than humans are not particularly limited, and various livestock, poultry, pets, laboratory animals, etc. can be targeted. Specifically, examples thereof include, but are not limited to, pigs, cattle, horses, sheep, goats, chickens, ducks, ostriches, domestic ducks, dogs, cats, rabbits, hamsters, mice, rats, and monkeys, and examples thereof include those who feel that their immune function is reduced, elderly people, and those who want to activate their immune function. Furthermore, even those who do not have any particular concerns etc. about their immune function can ingest it daily for the purpose of activating the immune function etc.

Furthermore, the present technology may be used for therapeutic purposes or non-therapeutic purpose uses. "Non-therapeutic use" is a concept that does not include medical practice, that is, treatment actions on the human body. For example, health promotion etc. is included.

When administering or ingesting the microorganisms of the present invention or a composition containing the microorganisms as an active ingredient (the composition of the present invention described above), the dosage or intake amount is appropriately selected depending on the age, weight, and health condition of the subject, the type of composition (pharmaceutical product, food and/or beverage, etc.), the form of the active ingredient (for example, live bacteria, dead bacterial cells, bacterial cell components, or culture), and the like.

The content or amount used of the microorganisms (live bacteria or dead bacterial cells) of the present invention is not particularly limited, but is 1 × 10⁴ to 1 × 10¹², more preferably 1 × 10⁶ to 1 × 10¹², and still more preferably 1 × 10⁸ to 1 × 10¹², in the composition. Note that the unit is CFU/g or CFU/mL, or cells/g or cells/mL. CFU stands for Colony forming unit.

The daily dose of the microorganisms (live bacteria or dead bacterial cells) of the present invention is not particularly limited, but is preferably 1 × 10⁴ to 1 × 10¹² per day, and may be 1 × 10⁶ to 1 × 10¹², or even 1 × 10⁸ to 1 × 10¹². Note that the unit is CFU/day or cells/day.

The combinations of upper and lower limits of these numerical ranges are only examples, and their combinations can be appropriately changed. When the active ingredient is a component or culture of microorganisms, for example, the amount of the component or culture can be an amount corresponding to the amount of bacterial cells.

The dosage or intake amount of the microorganisms of the present invention or the composition containing the microorganisms as an active ingredient (the composition of the present invention described above) is as described above; however, they may be administered or ingested once or multiple times (for example, twice or three times) per day. The period of administration or ingestion can be discontinued depending on the state of the immune function, or they may be administered or ingested continuously without discontinuation. As for "continuous", daily continuation or continuation at intervals is possible, but daily continuation of administration or ingestion of the microorganisms of the present invention or the composition containing the microorganisms as an active ingredient is preferable from the viewpoint of effects.

### [Examples]

Hereinafter, the present invention will be described more specifically based on Examples, but the present invention is not limited to the following Examples. In this Example, microorganisms belonging to a new species of the genus *Akkermansia* were isolated using the following methods, and the properties of these microorganisms were analyzed.

### (Method for Isolating Microorganisms)

At the Otsu Nutraceuticals Research Institute of Otsuka Pharmaceutical Co., Ltd., (Otsu City, Shiga Prefecture, Japan), fecal samples were collected from five adults belonging to the institute, and the collected fecal samples were transferred to an anaerobic chamber as soon as possible. The feces were suspended in physiological saline solution supplemented with cysteine to a concentration of 0.5 g/L, serially diluted, inoculated into Mucin Medium (composition is shown in Table 1 below), and liquid-cultured in an anaerobic chamber at 37°C (cultured for up to 7 days).

**[Table 1]**

| Mucin Medium | |
|---|---|
| KH₂PO₄ | 0.4 g/L |
| Na₂HPO₄ | 0.53 g/L |
| NH₄Cl | 0.3 g/L |
| NaCl | 0.3 g/L |
| MgCl₂ · ₆H₂O | 0.1 g/L |
| CaCl₂ | 0.11 g/L |
| alkaline trace element solution^{*1} | 1 ml/L |
| acid trace element solution^{*2} | 1 ml/L |
| resazurin | 0.5 mg/L |
| NaHCO₃ | 4 g/L |
| Na₂S·₉H₂O | 0.25 g/L |
| commercial hog gastric mucin (Type II; Sigma) | 0.5 % (w/v) |

| *1: alkaline trace element solution | |
|---|---|
| Na₂SeO₃ | 0.1 mM |
| Na₂WO₄ | 0.1 mM |
| Na₂MoO₄ | 0.1 mM |
| NaOH | 10 mM |

| *2: acid trace element solution | |
|---|---|
| FeCl₂ | 7.5 mM |
| H₃BO₄ | 1 mM |
| ZnCl₂ | 0.5 mM |
| CuCl₂ | 0.1 mM |
| MnCl₂ | 0.5 mM |
| CoCl₂ | 0.5 mM |
| NiCl₂ | 0.1 mM |
| HCl | 50 mM |

The culture solution in which the growth of microorganisms was confirmed was serially diluted with Mucin Medium, applied to a Mucin Medium agar plate, and anaerobically cultured at 37°C. One culture plate was prepared for each stool sample from one subject. Then, colonies were picked up as much as possible from each plate, seeded again into Mucin Medium, and anaerobically cultured. After adding glycerol to the culture solution in which growth was observed to a final concentration of 10%, 1 ml aliquots were dispensed and stored at -80°C to obtain stocks.

### (Sequencing of 16S rRNA Gene of Microorganisms)

A portion of the culture solution obtained during the preparation of the stock solution was subjected to heat treatment at 98°C for 15 minutes to extract DNA. Using the DNA as a template, an 800 bp DNA region encoding partial 16S rRNA was amplified using Bacterial 16S rRNA PCR Kit Fast (manufactured by Takara Bio Inc.), and then the sequence of the region was determined by Sanger sequencing (ABI PRISM 3500xL Genetic Analyzer System). The determined sequences were subjected to 16S rRNA phylogenetic analysis using NCBI BLAST (https://blast.ncbi.nlm.nih.gov/Blast.cgi) to select microbial strains classified in the genus *Akkermansia* (WON2089, WON2090, and WON2093). These three microbial strains were isolated from three different subjects.

Furthermore, at TechnoSuruga Laboratory Co., Ltd. (Shizuoka City, Shizuoka Prefecture, Japan), the almost full-length sequences of 16S rRNA of these microbial strains were determined by the following procedure.
- Using DNA extracted from the microbial strains as a template, the coding region of 16S rRNA was amplified by PCR using universal primers 9F and 1510R.
- The obtained PCR product was purified and then subjected to sequence analysis using 9F/515F/536R/926R/1099F/1510R primers and the ABI PRISM 3500xL Genetic Analyzer System to determine their sequences.

Furthermore, the full-length sequences thus obtained were subjected to BLAST+ analysis against the rRNA/ITS reference strain database of NCBI to calculate the homology etc. with microbial strains belonging to the known genus *Akkermansia.*

### (Creation of Phylogenetic Tree)

Furthermore, the 16S rRNA sequences of microbial strains belonging to the known genus *Akkermansia (A. muciniphila* and A. *glycaniphila)* and representative strains belonging to the phylum *Verrucomicrobia* were obtained from the NCBI database, and a phylogenetic tree was created using MEGA version 10.2.5 by the neighbor-joining method (bootstrap 1000 replicates).

### (Genome Analysis of Microorganisms)

The microbial strain (WON2089) was seeded into a medium (containing Trypticase Soy Broth (Becton, Dickinson and Company), 2 g/L N-acetyl-D-glucosamine (manufactured by Wako Pure Chemical Industries, Ltd.), 4 g/L threonine (manufactured by Wako Pure Chemical Industries, Ltd.), and 0.05% cysteine (manufactured by Wako Pure Chemical Industries, Ltd.)) and cultured at 37°C for 24 hours under anaerobic conditions.

DNA was extracted from the obtained culture using NucleoBond (registered trademark) HMW DNA (manufactured by Takara Bio Inc.). A volume of 2000 ng of the DNA was treated with Short Read Eliminator (manufactured by Circulomics Inc.) to remove low molecular weight DNA of approximately 10 kbp or less. This low molecular weight removal operation was repeated twice to prepare genomic DNA.

From the obtained genomic DNA (400 ng), a library for nanopore sequencing was prepared using a Ligation Sequencing Kit (manufactured by Nanopore Technologies). Then, sequence analysis (long-read sequence analysis) was performed using a MinION Flow Cell FLO-MIN106 R9.41 revD.

Furthermore, from the genomic DNA (500 ng), a library was prepared according to the protocol thereof using an Illumina NextEra DNA Flex Library Prep Kit (manufactured by Illumina, Inc.). Then, PhiX was added to a final concentration of 15%, and 155 bp × 2 sequence analysis (short-read sequence analysis) was performed using MiSeq.

Low-quality sequences were removed from the long reads and short reads thus obtained under the following conditions.
- Short reads with Q30 or less and long-read sequences with Q10 or less were removed.
- Short reads with 20 bp or less and long reads with 3,000 bp or less were removed.
- Remaining adapter sequences were removed.

Then, the sequencing data were assembled using Flye version 2.7 (https://www.nature.com/articles/s41587-019-0072-8) and Unicycler version 0.4.8 (https://pubmed.ncbi.nlm.nih.gov/28594827/) to obtain one circular sequence. Furthermore, Pilon version 1.23 (https://pubmed.ncbi.nlm.nih.gov/25409509/) was used to correct any misassemblies in the circular sequence to obtain a final single circular sequence.

The GC content was calculated from the circular sequence thus obtained. Furthermore, analysis was performed using DFAST (https://pubmed.ncbi.nlm.nih.gov/29106469/) to obtain the ANI (Average Nucleotide Identity) value.

### (Observation of Cell Morphology of Microorganisms)

The microbial strains (WON2089, WON2090, and WON2093) were seeded onto agar plates (containing modified GAM (manufactured by Nissui Pharmaceutical Co., Ltd.) and 0.1% mucin) and cultured at 37°C for 48 hours under anaerobic conditions. The colonies formed were scraped off, smeared on slide glasses, and Gram-stained using Faber G "Nissui" (manufactured by Nissui Pharmaceutical Co., Ltd.). Then, morphological observation was performed with an optical microscope.

### (Assimilability)

The microbial strains (WON2089, WON2090, and WON2093) were seeded onto agar plates (containing modified GAM and 0.1% mucin) and cultured at 37°C for 48 hours under anaerobic conditions. The colonies formed were scraped off with cotton swabs and suspended in medium attached to an Anaerobic Bacterial Biochemical Identification Kit API 20A (manufactured by bioMérieux Japan Ltd.). The cells were cultured for 48 hours under anaerobic conditions (N₂:CO₂:H₂/90:5:5), and acid production was determined.

Separately, after suspending colonies scraped off with cotton swabs in sterile physiological saline, enzyme activity was determined using an enzyme activity test kit API ZYM according to the instructions thereof (http://products.sysmex-biomerieux.net/product/pdf/25207E).

### (Fatty Acid Measurement)

The microbial strains (WON2089, WON2090, and WON2093) were seeded onto agar plates (containing modified GAM and 0.1% mucin) and cultured at 37°C for 48 hours under anaerobic conditions. The colonies formed were scraped off and freeze-dried. Then, fatty acids were measured using a bacterial fatty acid composition analysis system Sherlock Microbial Identification System (Version 6.0) according to the manual thereof.

### (Determination of Optimum Growth Temperature and pH)

The microbial strain (WON2089) was seeded into a liquid medium (containing modified GAM and 0.1% mucin) and cultured for 120 hours under anaerobic conditions. In the culture, the growth temperature was adjusted in the range of 10 to 50°C and the pH was adjusted in the range of 4.5 to 10 to carry out the test.

### (Ligand Assay)

The microbial strains (WON2089, WON2090, and WON2093) were seeded into a medium (containing Trypticase Soy Broth (manufactured by Becton, Dickinson and Company), 2 g/L N-acetyl-D-glucosamine (manufactured by Wako Pure Chemical Industries, Ltd.), 4 g/L threonine (manufactured by Wako Pure Chemical Industries, Ltd.), and 0.05% cysteine (manufactured by Wako Pure Chemical Industries, Ltd.)) and cultured at 37°C for 24 hours under anaerobic conditions. The cells were centrifuged at 10,000 g for 5 minutes at room temperature to remove the medium, the bacterial cells were washed with Otsuka Normal Saline, centrifuged again at 10,000 × g for 5 minutes at room temperature, and collected. Then, the cells were suspended in Otsuka Normal Saline so that the concentration was 10⁷ microorganisms/20 µl and heat-treated at 70°C for 30 minutes. The number of microorganisms was measured by a fluorescent staining method.

On the other hand, the following human embryonic kidney cell (HEK) transformants were maintained in a culture medium (containing DMEM (manufactured by Thermo Fisher Scientific), 50 U/ml penicillin (manufactured by Thermo Fisher Scientific), 50 µg/ml streptomycin (manufactured by Thermo Fisher Scientific), HEK-Blue Selection (manufactured by InvivoGen), and 10% FBS (manufactured by Thermo Fisher Scientific)) at 37°C under 5% CO₂.
HEK-Blue hTLR2 (manufactured by InvivoGen, hkbhtlr2),
HEK-Blue hTLR4 (manufactured by InvivoGen, hkbhtlr4), and
HEK-Blue hNOD2 (manufactured by InvivoGen, hkbhnod2).

These HEK transformants have stably integrated various pattern recognition receptor (PRR) genes (hTLR2, hTLR4, and hNOD2) and a reporter gene (SEAP). In such transformants, the expression of SEAP is promoted along with the activation of the PRR in the presence of a PRR agonist. Therefore, by using the color (absorbance) of a reagent (Quanti-Blue Solution), which changes according to the expression, as an index, the PRR agonistic activity of the microbial strains can be evaluated.

The cells thus maintained were adjusted with the culture medium to 2.0 × 10⁵ cells/ml, and 180 µl aliquots were seeded into a 96-well plate. After seeding the cells, bacterial cells adjusted to 10⁵ microorganisms/20 µl, 10⁶ microorganisms/20 µl, or 10⁷ microorganisms/20 µl were added to the 96-well plate in 20 µl aliquots and cultured for 24 hours. Pam3CSK4 (TLR2 agonist, manufactured by FUJIFILM Wako Pure Chemical Corporation), LPS from *Escherichia coli* O111:B4 (TLR4 agonist, manufactured by Sigma), and MDP (NOD2 agonist, manufactured by InvivoGen) were used as positive controls. A volume of 180 µl of Quanti-Blue Solution (manufactured by InvivoGen) was added to 20 µl of the obtained cell culture solution, and after reacting at 37°C for 1 hour, the absorbance at 620 nm was measured.

### (IgA Measurement)

First, Peyer's patch (PP) cells were prepared. Specifically, first, mice (BALB/c CrSlc/male, 9 to 13 weeks old) were bred and maintained by ad libitum intake of Solid Feed MF and tap water. After euthanizing the mice after quarantine by cervical dislocation, the small intestine was removed. After washing the outside of the intestinal tract with physiological saline, the PP cut out from the outer surface of the small intestine with ophthalmological scissors was placed in 24-well plates to which RPMI 1640 had been added and cooled on ice. The cells were made single using a stainless steel mesh and washed well with 5 mL of complete medium (+) (RPMI 1640 medium supplemented with 50 µM mercaptoethanol, 100 U/mL penicillin, 100 µg/mL streptomycin, 15 mM HEPES (pH 7.2), and 10% FBS). The cell suspension was filtered through a 40 µm cell strainer and centrifuged at 280 × g for 10 minutes at 4°C. After centrifugation, the culture supernatant was removed by suction, and the cells were resuspended in 5 mL of complete medium (+). After repeating the same operation twice, the obtained precipitate was suspended in 1.0 mL of complete medium (+) and adjusted so that the cell concentration was 1.2 × 10⁶ cells/mL. A volume of 200 µL of the cell suspension thus prepared was seeded into each well of 96-well plates, and the cells were cultured at 37°C under 5% CO₂ for about 2 hours until the PP cells settled to the bottom of the plate.

Next, the microbial strain (WON2089) was cultured by the method described in (Ligand Assay) above, the culture was subjected to heat treatment, and adjusted to 10⁷ microorganisms/20 µl. Then, 20 µL of the dead bacteria solution thus obtained was added to the PP cells and cultured at 37°C under 5% CO₂ for 6 days. After culturing, the plate was centrifuged at 1,000 rpm (180 × g) for 10 minutes using a centrifuge (KUBOTA 5200), 150 µL of the supernatant was collected in a microtube, and stored frozen at -80°C until IgA measurement.

Then, IgA measurement was performed using an IgA, Mouse, ELISA Quantitation Kit (manufactured by Bethyl Laboratories).

### (Results)

As described above, colony formation of microbial strains belonging to the genus *Akkermansia* (WON2089, WON2090, and WON2093) could be detected from the feces of 3 out of 5 subjects. Furthermore, as a result of determining and comparing the 16S rRNA sequences of these microbial strains, it was clarified that all of them carry the 16S rRNA gene composed of the same sequence.

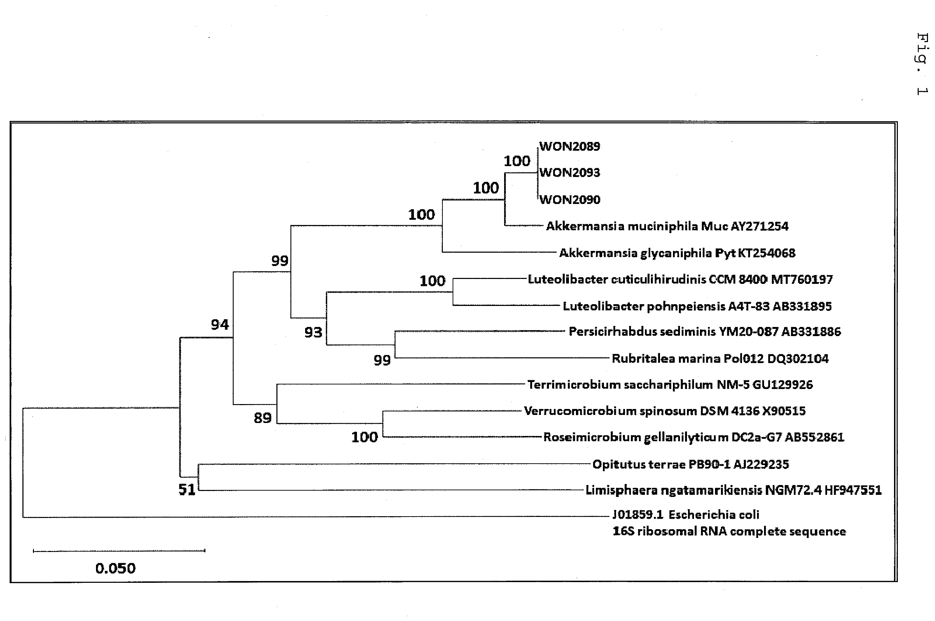

As a result of BLAST+ analysis of the 16S rRNA sequence of the microorganism against the rRNA/ITS reference strain database of NCBI, as shown in Table 2 below, the homology with *A. muciniphila* was 98.0%, and the homology with A. *glycaniphila* was 94.0%. Furthermore, as shown in the phylogenetic tree created based on the 16S rRNA sequences (Fig. 1), the three microbial strains isolated this time were microorganisms belonging to the genus *Akkermansia,* but it was suggested that they could be different bacterial species.

**[Table 2]**

| Registered Name | Strain Name | Accession No. | Homology (%) |
|---|---|---|---|
| Akkermansia muciniphila | Muc | AY271254 | 90.0 (1406/1435) |
| Akkermansia glycaniphila | Pyt | KT254068 | 94.0 (1355/1442) |
| Persicirhabdus sediminis | YM20-0E7 | AB331836 | 86.5 (1272/1471) |
| Luteolibacter algae | A5J-41-2 | AB331893 | 86.0 (1268/1475) |
| Luteolibacter pohnpeiensis | A4T-83 | AB331895 | 85.8 (1267/1476) |
| Haloferula sargassicola | MN1-1037 | AB372856 | 86.4 (1228/1421) |
| Luteolibacter gellanilyticus | CB-286403 | LN833280 | 86.6 (1212/1400) |
| Oceaniferula marina | N1E253 | MN897724 | 85.5 (1259/1472) |
| Haloferula harenae | YM23-227 | AB372852 | 86.3 (1217/1410) |
| Haloferula rosea | 06SJR1-1 | AB372853 | 85.9 (1216/1416) |
| Haloferula luteola | YC6886 | FJ032193 | 86.2 (1202/1395) |
| Rubritalea halochordaticola | MN1-1006 | AB543683 | 85.1 (1247/1465) |
| Rubritalea sabuli | YM29-052 | AB353310 | 85.1 (1247/1465) |
| Rubritalea tangerina | YM27-005 | AB297806 | 85.1 (1254/1473) |
| Haloferula phyci | AK18-024 | AB372854 | 85.7 (1213/1416) |
| Haloferula helveola | 05IJR53-1 | AB372855 | 85.7 (1212/1415) |
| Haloferula chungangensis | CAU1074 | JN001409 | 85.6 (1219/1424) |
| Luteolibacter luojiensis | CCTCC2010415 | JN630810 | 85.7 (1203/1403) |
| Rubritalea spongiae | YM21-132 | AB297805 | 84.7 (1248/1474) |
| Rubritalea squalenifaciens | HOact23 | AB277853 | 84.9 (1219/1436) |

For WON2089, whole genome sequence analysis was determined, and based on the sequence, ANI analysis was performed using DFAST. Then, when compared with the two bacterial strains of A. *muciniphila,* as shown in Table 3 below, the value was about 82.5% for each, which was lower than the current species cutoff value of 95% ANI. That is, it was indicated that the isolated microbial strain is a new species of *Akkermansia.*

**[Table 3]**

| organism_name | strain | accession | ANI |
|---|---|---|---|
| *Akkermansia muciniphila* | strain = DSM 22959 | GCA_008000975.1 | 82.5 |
| *Akkermansia muciniphila* | strain = ATCC BAA-835 | GCA_000020225.1 | 82.5 |
| *Akkermansia glycaniphila* | strain = Pyt | GCA_001683795.1 | 77.4 |

As a result of analyzing the GC content based on the whole genome sequence, it was 56.8 mol %. As shown in Table 4 below, the morphology of all the microbial strains (WON2089, WON2090, and WON2093) was spherical to short rod-shaped. In addition, all of the microbial strains were Gram-negative and oxidase-negative, but catalase-positive. Furthermore, as shown in Table 5 below, the optimum growth temperature of all the microbial strains was 30 to 40°C, while no growth was observed at 20°C or less or 50°C or more. Moreover, the optimum pH was 6.0 to 8.5, while no growth was observed at pH 5.0 or less or pH 10.0 or more. As for the GC content, cell morphology, Gram stainability, oxidase reaction, catalase reaction, and growth conditions, the properties were similar to those of known *Akkermansia.*

**[Table 4]**

| Agar medium used: Modified GAM + 0.1% mucin | | |
|---|---|---|
| Growth temperature: 37°C | | |
| Culture time: 72 hours | | |
| Evaluation item | | Results |
| Cell morphology Gram stainability | | Ovoid cocci - short rods (0.4-0.5 x 0.5-1.0 µm) - |
| Spore | | - |
| Motility | | - |
| Colony morphology | Diameter | 0.5-0.7 mm |
| | Color | Pale yellow |
| | Shape | Circular |
| Catalase reaction | | + |
| Oxidase reaction | | |

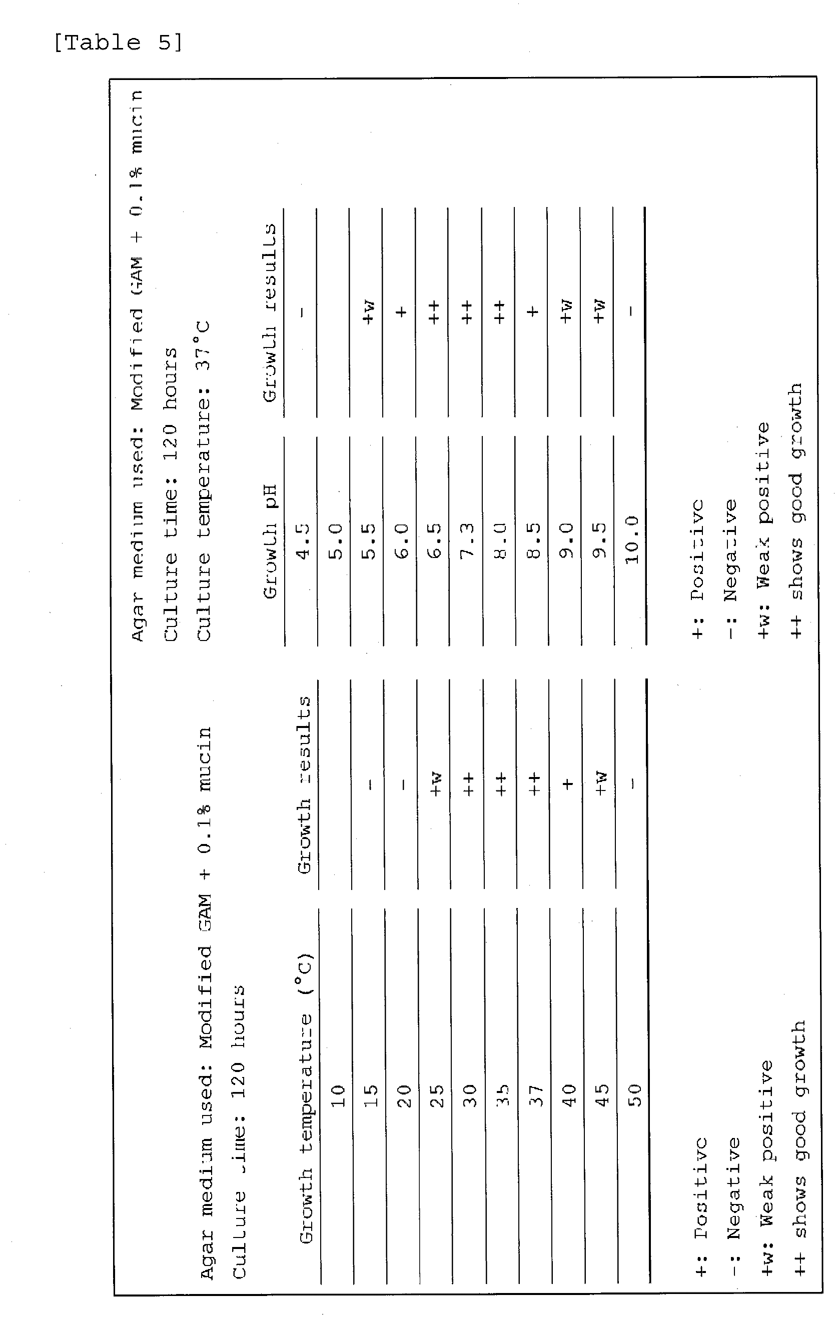

In the acid production test using API 20A, as shown in Table 6 below, all of the microbial strains (WON2089, WON2090, and WON2093) showed growth on D-glucose, lactose, and D-mannose, as in the two known *Akkermansia* species (*A*. *muciniphila* (strain ATCC BAA-835) and A. *glycaniphila* (strain DSM100705)). On the other hand, unlike the two known species, growth on D-mannitol was also observed in all of the microbial strains. Furthermore, the growth on maltose, which was observed in *A. glycaniphila,* was not observed in any of the microbial strains, as in A. *muciniphila.*

**[Table 6]**

| Acid production by API 20A | | | | | |
|---|---|---|---|---|---|
| Sugar | WON2089 | WON2090 | WON2093 | A. muciniphila BAA835 | A. glycaniphila DSM 100705 |
| D-Glucose | + | + | + | + | + |
| D-Mannitol | + | + | + | - | - |
| Lactose | + | + | + | + | + |
| Sucrose | - | - | - | - | - |
| Maltose | - | - | - | - | + |
| Salicin | - | - | - | - | - |
| D-Xylose | - | - | - | - | - |
| L-Arabinose | - | - | - | - | - |
| Glycerin | - | - | - | - | - |
| D-Cellobiose | - | - | - | - | - |
| D-Mannose | + | + | + | + | + |
| D-Melezitose | - | - | - | - | - |
| D-Raffinose | - | - | - | - | - |
| D-Sorbitol | - | - | - | - | - |
| L-Rhamnose | - | - | - | - | - |
| D-Trehalose | - | - | - | - | - |

As a result of the enzyme activity test using API ZYM, the enzyme activities shown in Table 7 below were observed for WON2089.

**[Table 7]**

| Bacterial enzyme activity by APIZYM | |
|---|---|
| Enzyme | WON2089 |
| Alkaline phosphatase | - |
| Esterase (C4) | - |
| Esterase lipase (C8) | - |
| Lipase (C14) | - |
| Leucine arylamidase | - |
| Valine arylamidase | - |
| Cysteine arylamidase | - |
| Trypsin | - |
| Chymotrypsin | - |
| Acid phosphatase | - |
| Naphthol-AS-BI-phosphohydrolase | + |
| α-Galactosidase | - |
| β-Galactosidase | + |
| β-Glucuronidase | + |
| α-Glucosidase | - |
| β-Glucosidase | - |
| N-Acetyl-β-glucosaminidase | + |
| α-Mannosidase | - |
| α-Fucosidase | - |

As a result of measuring the fatty acids of the bacterial cells of WON2089 and two known *Akkermansia* species (*A. muciniphila* and *A*. *glycaniphila*)*,* the cellular fatty acid compositions shown in Table 8 below were observed.

**[Table 8]**

| Fatty Acid Type | WON2089 | Akkermansia muciniphila | Akkermansia glycaniphila |
|---|---|---|---|
| Saturated Fatty Acid | | | |
| C_{13:0} | 0.1 | 0.3 | 0.1 |
| C_{14:0} | 0.6 | 0.7 | 1.0 |
| C_{15:0} | 4.1 | 11.9 | 23.8 |
| C_{16:0} | 4.0 | 5.6 | 5.1 |
| C_{17:0} | 2.7 | 4.1 | 0.2 |
| C_{18:0} | 1.0 | 0.7 | 0.3 |
| C_{14:0} DMA | 0.3 | 0.1 | 0.2 |
| C_{16:0} aldehyde | 0.6 | 0.7 | 0.6 |

| Unsaturated Fatty Acid | | | |
|---|---|---|---|
| C_{16:1} ω7c | - | 0.1 | 0.1 |
| C_{18:1} ω9c | 5.8 | 3.1 | 1.5 |
| C_{18:2} ω6,9c | 0.5 | 0.4 | 0.2 |

| Branched Fatty Acid | | | |
|---|---|---|---|
| C_{11:0} anteiso | - | - | 0.1 |
| C_{13:0} anteiso | 0.1 | - | - |
| C_{14:0} iso | 5.6 | 10.1 | 4.0 |
| C_{15:0} anteiso | 55.5 | 45.4 | 44.5 |
| C_{15:0} iso | 1.5 | 1.3 | 1.9 |
| C_{16:0} iso | 2.7 | 3.6 | 1.8 |
| C_{17:0} anteiso | 1.8 | 0.9 | 0.2 |
| C_{17:0} iso | 0.3 | 0.1 | - |
| C_{18:0} iso | 0.1 | 0.1 | - |

| Hydroxy Fatty Acid | | | |
|---|---|---|---|
| C_{15:0} 3OH | 7.5 | 7.7 | 10.8 |
| C_{15:0} iso 3OH | 0.2 | - | 0.1 |
| C_{16:0} 3OH | 0.6 | 0.9 | 1.1 |
| C_{17:0} 3OH | 1.4 | 1.1 | 0.4 |
| C_{17:0} anteiso | 0.6 | 0.2 | 0.2 |

| Summed Features | | | |
|---|---|---|---|
| 1 | - | 0.1 | 0.1 |
| 5 | 1.1 | - | 1.1 |
| 6 | 0.4 | 0.1 | 0.2 |
| 9 | 0.6 | 0.6 | 0.4 |
| 10 | 0.4 | 0.3 | 0.1 |
| 11 | 0.2 | 0.1 | 0.1 |
| Total | 100 | 100 | 100 |

Analysis according to the cellular fatty acid composition analysis manual of the Sherlock Microbial Identification System (Version 6.0, MIDI, USA)
Agar medium used: Modified GAM + 0.1% mucin
   *Fatty acids for which the fatty acid species cannot be specified due to almost the same retention time are expressed as the following Summed Feature:
      Summed Feature 1: C13:1 at 12-13, C14:0 aldehyde and C11:1 2OH
      Summed Feature 2: C12:0 3OH and C13:0 DMA
      Summed Feature 3: C15:0 ISO aldehyde and unknown 13.570
      Summed Feature 4: unknown 14.762, C15:2 FA, C15:2 and C15:1 ω8c
      Summed Feature 5: C15:0 DMA and C14:0 3OH
      Summed Feature 6: C15:0 anteiso 3OH and C16:1 ω9c DMA
      Summed Feature 7: C17:2 at 16,760 and C17:1 ω9c
      Summed Feature 8: C17:1 ω8c and C17:2 at 16.801
      Summed Feature 9: C16:0 iso 3OH and unknown 17.157 DMA
      Summed Feature 10: C18:1 ω7c and unknown 17.834
      Summed Feature 11: C17:0 iso 3OH and C18:2 DMA
      Summed Feature 12: unknown 18.622 and C19:0 iso
      Summed Feature 13: 15:0 anteiso DMA and 14:0 2OH

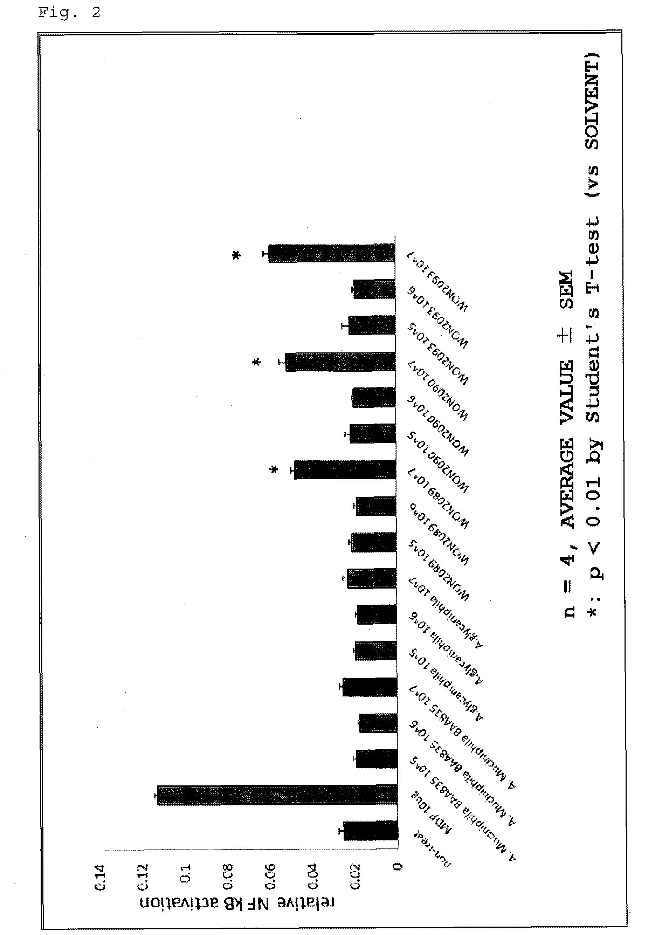

As a result of analyzing ligand activity, as shown in Fig. 2, NOD2 agonistic activity was not observed for the two known *Akkermansia* species (*A. muciniphila* (strain ATCC BAA-835) and A. *glycaniphila* (strain DSM100705)), but the activity was observed in all of the microbial strains (WON2089, WON2090, and WON2093). Although not shown in the figure, TLR2 agonistic activity and TLR4 agonistic activity were observed in both the two known species and the microbial strains; however, the TLR2 agonistic activity of the microbial strains was about 1.5 times higher than that of the two known species. The TLR4 agonistic activity of the microbial strains was slightly higher than that of the two known species.

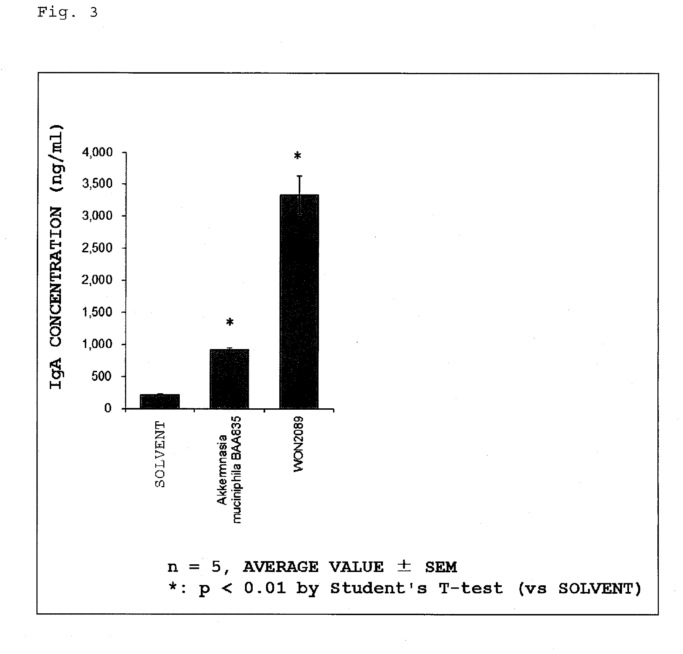

Furthermore, regarding WON2089, the IgA secretion-promoting effect thereof was evaluated, and as shown in Fig. 3, it was clarified that WON2089 has this effect; it was also clarified that the effect is at least twice as high as that of known A. *muciniphila* (strain ATCC BAA-835) .

### [Industrial Applicability]

As described above, the microorganisms belonging to the genus *Akkermansia* of the present invention are new species of microorganisms that differ from known *Akkermansia* at least in their assimilability to D-mannitol. Further, the microorganisms belonging to the genus *Akkermansia* of the present invention have NOD2 agonistic activity, which is not found in known *Akkermansia,* and may also have a high IgA secretion-promoting effect.

Therefore, the microorganisms belonging to the genus *Akkermansia* of the present invention can activate the host's immunity etc. via activation of NOD2 etc. Therefore, they are useful as pharmaceuticals, foods, etc. having such an activating effect.

### [Accession Number]

(1) Identification label: WON2089
(2) Accession number: NITE BP-03622
(3) Deposit date: March 17, 2022
(4) Depositary institution: NITE Patent Microorganisms Depositary of the National Institute of Technology and Evaluation
   (1) Identification label: WON2090
   (2) Accession number: NITE BP-03623
   (3) Deposit date: March 17, 2022
   (4) Depositary institution: NITE Patent Microorganisms Depositary of the National Institute of Technology and Evaluation
      (1) Identification label: WON2093
      (2) Accession number: NITE BP-03624
      (3) Deposit date: March 17, 2022
      (4) Depositary institution: NITE Patent Microorganisms Depositary of the National Institute of Technology and Evaluation

| | | |
|---|---|---|
| 0-1 | Form PCT/RO/134 | |
| | Indications Relating to Deposited Microorganism(s) or other Biological Material (PCT Rule 13bis) | |
| 0-1-1 | Prepared Using | JPO-PAS |
| | | i480 |
| 0-2 | International Application No. | |
| 0-3 | Applicant's or agent's file reference | IBPF23-505WO |
| | | |
| 1 | The indications made below relate to the deposited microorganism(s) or other biological material referred to in *the description on:* | |
| 1-1 | Paragraph Number | 0045 |
| 1-3 | Identification of deposit | |
| 1-3-1 | Name of depositary institution | NPMD NITE Patent Microorganisms Depositary of the National Institute of Technology and Evaluation (NPMD) |
| 1-3-2 | Address of depositary institution | Room 122, 2-5-8 Kazusa-Kamatari, Kisarazu, Chiba 292-0818, Japan |
| 1-3-3 | Date of deposit | March 17, 2022 (17.03. 2022) |
| 1-3-4 | Accession Number | NPMD NITE BP-03622 |
| 1-5 | Designated States for Which Indication are Made | All designations |
| 2 | The indications made below relate to the deposited microorganism(s) or other biological material referred to in the description on: | |
| 2-1 | Paragraph Number | 0046 |
| 2-3 | Identification of deposit | |
| 2-3-1 | Name of depositary institution | NPMD NITE Patent Microorganisms Depositary of the National Institute of Technology and Evaluation (NPMD) |
| 2-3-2 | Address of depositary institution | Room 122, 2-5-8 Kazusa-Kamatari, Kisarazu, Chiba 292-0818, Japan |
| 2-3-3 | Date of deposit | March 17, 2022 (17.03. 2022) |
| 2-3-4 | Accession Number | NPMD NITE BP-03623 |
| 2-5 | Designated States for Which Indication are Made | All designations |
| 3 | The indications made below relate to the deposited microorganism(s) or other biological material referred to in the description on: | |
| 3-1 | Paragraph Number | 0047 |
| 3-3 | Identification of deposit | |
| 3-3-1 | Name of depositary institution | NPMD NITE Patent Microorganisms Depositary of the National Institute of Technology and Evaluation (NPMD) |
| 3-3-2 | Address of depositary institution | Room 122, 2-5-8 Kazusa-Kamatari, Kisarazu, Chiba 292-0818, Japan |
| 3-3-3 | Date of deposit | March 17, 2022 (17.03. 2022) |
| 3-3-4 | Accession Number | NPMD NITE BP-03624 |
| 3-5 | Designated States for Which Indication are Made | All designations |

## Claims

1. A microorganism belonging to the genus *Akkermansia,*
having a 16S rRNA gene comprising a nucleotide sequence having homology of 98.0% or less to a nucleotide sequence set forth in SEQ ID NO: 1, homology of 94.0% or less to a nucleotide sequence set forth in SEQ ID NO: 2, or homology of more than 98.0% to a nucleotide sequence set forth in SEQ ID NO: 3, and
having the following properties 1) to 4):
1) having NOD2 agonistic activity,
2) having assimilability to D-mannitol,
3) being a Gram-negative, anaerobic bacterium with a spherical to short rod-shaped form, and
4) being negative in an oxidase test and positive in a catalase test.

2. A microorganism specified by accession number NITE BP-03622, NITE BP-03623 or NITE BP-03624.

3. The microorganism according to claim 1, which further has an IgA secretion-promoting effect at least twice as high as that of type strain ATCC BAA-835 of *Akkermansia muciniphila.*

4. A composition for activating immunity comprising the microorganism according to any one of claims 1 to 3 as an active ingredient.

5. A composition for activating NOD2 comprising the microorganism according to any one of claims 1 to 3 as an active ingredient.

6. A composition for promoting the secretion of IgA comprising the microorganism according to claim 2 or 3 as an active ingredient.
